# EUROPEAN PATENT SPECIFICATION

(11) **EP 2 682 886 B1**
(45) Date of publication and mention of the grant of the patent: **23.03.2016**
(21) Application number: 13175438.4
(22) Date of filing: 05.07.2013
(51) Int. Cl.: G06F 19/00, H04L 12/28, H04W 84/10

(54) **System for Patient-staff Acknowledgment, Wireless Staff member Device, Wireless Patient member Device and Patient-staff Acknowledgment Method**
System zur Patient-Personal-Bestätigung, drahtlose Vorrichtung für Personalangehörigen, drahtlose Vorrichtung für Patientenangehörigen und Patient-Personal-Bestätigungsverfahren
Système pour accusé de réception patient-personnel, dispositif sans fil pour membre du personnel, dispositif sans fil patient et procédé d'accusé de réception patient-personnel

(30) Priority: 05.07.2012 US 201261668066 P; 28.08.2012 SE 1250956; 14.09.2012 SE 1251027
(43) Date of publication of application: 08.01.2014
(73) Proprietor: CMT Captera MedTech AB, 24643 Löddeköpinge (SE)
(72) Inventor: Jostrand, David, 234 34 Lomma (SE); Verstraelen, Hans, 6077CV St.Odilienberg (NL)
(74) Representative: KIPA AB

(56) References cited:
- WO-A2-2010/102069
- WO-A2-2011/124993
- US-A- 5 537 459

## Description

### BACKGROUND OF THE INVENTION

### Field of the Invention

This disclosure pertains in general to the field of patient monitoring. More particularity, the disclosure relates to a wireless system comprising a staff member device and a patient member device and even more particularly to cancellation of a patient alarm.

### Description of the Prior Art

There are some patient monitoring and patient alarm systems known from prior art. As an example, the prior art document US2011/0211563 A1 discloses a location tracking system, which includes handling of patient alarms.

However, in this document, only the staff or nurses are able to trigger an alarm.

Furthermore, from paragraph [0022] of the document, it is clear that signals between a server and a plurality of user equipment can be routed through a plurality of base stations. The network can be based on Bluetooth. However, from this document it is not clear how the routing is performed. Furthermore, the document does not disclose anything about any redundancy in the system in order to make it more reliable. Moreover, a system, such as the system disclosed in the prior art document, may have problems with dead-spots, i.e. locations where no signal can be obtained.

Hence, an improved patient monitoring system would be advantageous. In addition, a system with optimized routing of signals may be advantageous. Furthermore, a more reliable patient monitoring system may be advantageous. Moreover, a patient monitoring system without dead-spots, i.e. locations where no signal can be obtained, may be advantageous.

WO2010/102069 A2 discloses that a monitoring device has a processor, which controls the information appearing on a display. A detector determines the physical presence of a clinician token within a detection area in the vicinity of the medical patient monitoring device. The clinician token indicates the identity of a clinician. The processor takes a first predetermined action in response to detection of the clinician token. The first predetermined action is associated with the identity of the clinician.

However, although the system of this document may send alarms, patient alarms cannot be handled.

There are also some other patient monitoring and patient alarm systems known from prior art.

However, in the methods of the prior art, the cancellation of an alarm is done on a room level. Thus, it is possible that several patients in the same room have sent an alarm and the alarm of that room has been cancelled, but only one patient in the room has been attended to.

Thus, there may be a need for a new patient monitoring or patient alarm system, wherein also patients are able to send an alarm. In particular, there may be a need for a new patient monitoring or patient alarm system, wherein patient devices for sending alarms are wireless.

Furthermore, an improved system, wherein the cancellation of an alarm is done on a personal level would be advantageous.

### SUMMARY OF THE INVENTION

Accordingly, examples of the present disclosure preferably seek to mitigate, alleviate or eliminate one or more deficiencies, disadvantages or issues in the art, such as the above-identified, singly or in any combination by providing a system, a staff member device, a patient member device and a method for patient monitoring according to the appended patent claims.

According to aspects of the disclosure, a system for patient-staff acknowledgment, a wireless staff member device, a wireless patient member device and a method of patient-staff acknowledgment are disclosed, whereby the cancellation of an alarm is done on a personal level, i.e. a cancellation is performed for only one patient, instead of for a room. The system may be a network system, a patient monitoring system or a patient alarm system. The system may also comprise a staff alarm system. The wireless patient member device may also be referred to as a wireless patient-specific device and the wireless staff member device may be referred to as a wireless staff member-specific device.

According to one aspect of the disclosure, a system for patient-staff acknowledgment is provided. The system includes a wireless staff member device. The wireless staff member device includes a first receiver for receiving a first signal from a wireless patient member device. The wireless staff member device also includes a first transmitter for transmitting a second signal to the wireless patient member device. Furthermore, the wireless staff member device includes at least one first switch. The system includes a wireless patient member device. The wireless patient member device includes at least one second switch. Furthermore, the wireless patient member device includes a second transmitter for transmitting the first signal to the wireless staff member device. Moreover, the wireless patient member device includes a second receiver for receiving the second signal from the wireless staff member device.

According to another aspect of the disclosure, a wireless staff member device is provided. The wireless staff member device is for use in a system for patient-staff acknowledgment. The wireless staff member device includes a receiver for receiving a first signal from a wireless patient member device. Moreover, the wireless staff member device includes a transmitter for transmitting a second signal to the wireless patient member device. Furthermore, the wireless staff member device includes at least one switch. The wireless staff member device includes a control unit configured to, upon affecting one of the at least one switch, activate the receiver to receive a first signal within a range less than a threshold distance during a predetermined activation time, the control unit is further configured to transmit the second signal by the transmitter after receiving the first signal by the receiver within the predetermined activation time.

According to yet another aspect of the disclosure, a wireless patient member device for use in a system for patient-staff acknowledgment is provided. The patient member device includes at least one switch. Furthermore, the patient member device includes a transmitter for transmitting a first signal to a wireless staff member device. Moreover, the patient member device includes a receiver for receiving a second signal from the wireless staff member device. The patient member device includes a control unit configured to, upon affecting one of the at least one switch the first time, activate an alarm, the control unit is further configured to, upon affecting one of the at least one switch a second time, transmit the first signal by the transmitter and cancelling the alarm after receiving the second signal by the receiver as a response to the transmitted first signal.

According to a further aspect of the disclosure, a method of patient-staff acknowledgment is provided. The method includes activating a receiver of a wireless staff member device to receive a first signal within a threshold distance from a wireless patient device for a predetermined activation time, by affecting a switch of the wireless staff member device. Furthermore, the method includes activating a transmitter of the wireless patient member device to transmit the first signal by affecting a switch of the wireless patient device. This transmitter may be referred to as a second transmitter. Moreover, the method includes transmitting a second signal from a transmitter of the wireless staff member device when the receiver of the wireless staff member device is receiving the first signal within the predetermined activation time. This transmitter may be referred to as a first transmitter and the receiver may be referred to as a first receiver. The method includes cancelling an alarm activated by the wireless patient member device when the receiver of the wireless patient device is receiving the second signal. The receiver of the wireless patient device may be referred to as a second receiver.

Further examples of the disclosure are defined in the dependent claims, wherein features for the second and subsequent aspects of the disclosure are as for the first aspect mutatis mutandis.

Some examples of the disclosure provide for that cancellation of an alarm is performed on a personal level, not a room level.

Some examples of the disclosure also provide for accurate measurement of position.

Some examples of the disclosure also provide for that dead-spots, i.e. places where no signal can be obtained, are avoided.

Some examples of the disclosure also provide for that the patients and/or the staff members can move around freely and still have access to their devices.

Some examples of the disclosure also provide for a dual use of the switch such as button for cancelling an alarm on the wireless staff member device.

Some examples of the disclosure also provide for an extra safety feature for staff members.

Some examples of the disclosure also provide for traceability of alarms and/or cancellations of alarms.

Some examples of the disclosure also provide for avoidance of unnecessary running around of staff members, since the first staff member acknowledges the alarm and other staff members to not need to check the patient, who triggered the alarm.

Some examples of the disclosure also enable distinguishing between an emergency situation and a non-emergency situation.

Some examples of the disclosure also provide for redundancy in the system, and thus increased reliability of the system, since two gateways are used.

Some examples of the disclosure also provide for redundancy, and thus increased reliability of the system, since two different routes are selected and set-up.

Some examples of the disclosure also provide for that a route with the best signal quality is set-up. Thus, the system may be optimized.

Some examples of the disclosure also provide for that a redundant route with the second best signal quality is set-up. Thus, the system is made more reliable.

Some examples of the disclosure also provide for self-healing of the system, for example when a transceiver is replaced with a new transceiver.

Some examples of the disclosure also provide for that no physical device configuration or user intervention is needed during replacement of a transceiver.

It should be emphasized that the term "comprises/comprising" when used in this specification is taken to specify the presence of stated features, integers, steps or components but does not preclude the presence or addition of one or more other features, integers, steps, components or groups thereof.

### BRIEF DESCRIPTION OF THE DRAWINGS

These and other aspects, features and advantages of which examples of the invention are capable of will be apparent and elucidated from the following description of examples of the present invention, reference being made to the accompanying drawings, in which
Fig. 1 illustrates an area in which patient monitoring is performed;
Fig. 2 is an exemplary structure of a system;
Fig. 3 is a detailed view of a patient member device;
Fig. 4 is a detailed view of a staff member device;
Fig. 5 illustrates communication between some units of the system;
Fig. 6 illustrates a transceiver according to some examples; and
Fig. 7 is a signalling diagram illustrating acknowledgment signals and cancellation signals sent between units of the system; and
Fig. 8A and 8B is illustrating examples of a patient member device and a staff member device schematically.

### DESCRIPTION OF THE PREFERRED EMBODIMENTS

Specific examples of the disclosure will now be described with reference to the accompanying drawings. This disclosure may, however, be embodied in many different forms and should not be construed as limited to the examples set forth herein; rather, these examples are provided so that this disclosure will be thorough and complete, and will fully convey the scope of the disclosure to those skilled in the art. The terminology used in the detailed description of the examples illustrated in the accompanying drawings is not intended to be limiting of the disclosure. In the drawings, like numbers refer to like elements.

The following description focuses on an example of the present disclosure applicable to a system and in particular to a method of setting up a system and/or to acknowledgment and cancellation of alarms in such a system. However, it will be appreciated that the disclosure is not limited to these applications but may be applied to many other systems including for example monitoring of patients in their homes.

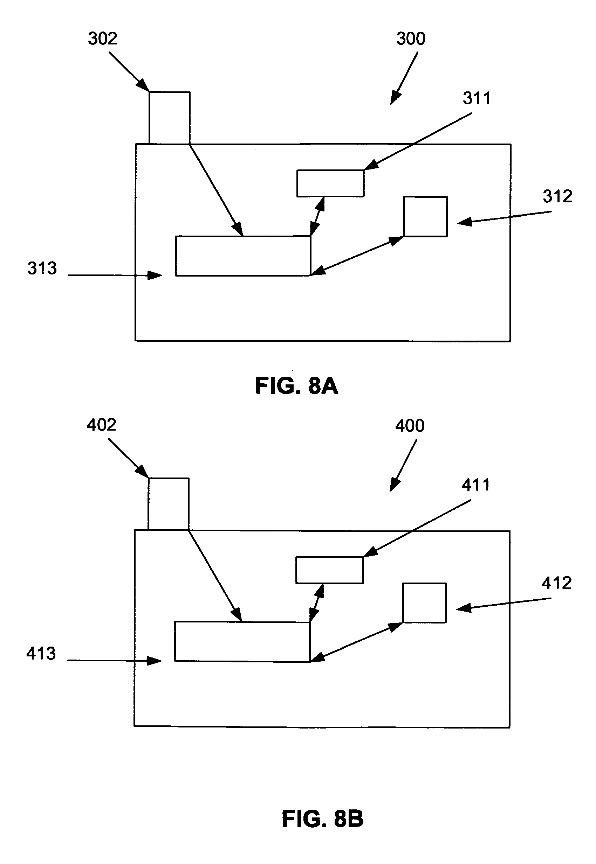

The disclosure relates to the mechanism of sending an alarms and/or requesting/obtaining acknowledgement and cancellation of the alarm. The device, system and method of the disclosure may be briefly described as, with referral to Fig. 8A and 8B:

Starting from a no-alarm state, upon activation of the wireless patient member device 300, by affecting a switch 302 of the wireless patient member device 300, an alarm may be activated. After activating the alarm and onwards the wireless patient member device 300 may be in alarm state.

Additionally, in some examples, by activating the alarm upon affecting of a switch 302 of the wireless patient member device 300, a transmitter 312 of the wireless patient member device may transmit an alarm signal to the nearest wireless network transceiver or, when the nearest wireless network transceiver is unknown, to a group of wireless network transceivers. The wireless network transceiver nearest to the wireless patient member device 300 may transmit an acknowledgement signal to the wireless patient member device 300. Additionally and/or alternatively, in some examples of the disclosure, the alarm signal transmitted to the network may either be with high priority or low priority depending on which switch was affected or for how long the switch 302 was hold.

Additionally, in some examples of the disclosure, the nearest wireless network transceiver acknowledging the alarm signal may be used to determine the position of the patient wearing or carrying the wireless patient member device 300.

While in alarm state, upon activation of a switch 302 of the wireless patient member device 300, the wireless patient member device 300 may transmit a first signal using the transmitter 312 of the wireless patient member device 300. This first signal may contain a request for cancellation of the alarm state. Additionally, in some examples of the disclosure, the transmittance level of the first signal may be reduced to only transmit the signal within a threshold distance. For example, such that only a wireless staff member device in close range is able to receive this signal.

By affecting a switch 402 of a wireless staff member device 400 a receiver 411 may be activated to receive the first signal for a limited time. Additionally, the range for receiving the first signal may be reduced so that the first signal may only be received within a threshold distance.

When the wireless staff member device 400 receives the first signal (the request for cancellation of the alarm state) from the wireless patient member device 300, it may transmit a second signal using the transmitter 412 to the receiver 311 of the wireless patient member device 300 to cancel the alarm state of wireless patient member device 300. Additionally, and/or alternatively, in some examples of the disclosure, an audible indication and/or a visible indication of a successful cancellation of an alarm may be used. The indication may be on the staff member device 400 and/or patient member device 300 and/or a network transceiver.

Additionally, in some examples of the disclosure, after the transmitter 412 of the wireless staff member device 400 has transmitted the second signal, the wireless staff member device 400 may transmit a cancellation signal to the nearest wireless network transceiver or, when the nearest wireless network transceiver is unknown, to a group of wireless network transceivers to cancel the alarm of the wireless patient member device 300. After receiving the cancellation signal a specific network transceiver (the network transceiver receiving the cancellation signal) returns an acknowledgement to the wireless staff member device 400.

Additionally, in some examples of the disclosure, if the wireless patient member device 300 does not receive a second signal from a wireless staff member device 400 to cancel its alarm state, one or more repeat transmission attempts may be made to transmit the first signal to a wireless staff member device 400 within range. Each attempt may be a first signal lasting for 1 to 10 seconds, such as 1 to 5 seconds, such as 1 or 2 seconds, etc. The number of attempts may be between 1 to 20 times, such as 1 to 10 times, such as 1 to 5 times, such as 1 or 2 times, such as between 10 to 20 times, such as between 10 to 15 times etc.

Additionally, in some examples of the disclosure, if no second signal from a wireless staff member device 400 to cancel the alarm state is received for a predetermined number of attempts, as a fall back safe method, the wireless patient member device 300 may transmit a repeated alarm signal at normal transmit level to the nearest wireless network transceiver or, when the nearest wireless network transceiver is unknown, to a group of wireless network transceivers. After receiving the repeated alarm signal a specific network transceiver (the network transceiver closest to the wireless patient member device 300 receiving the repeated alarm signal) returns an acknowledgement to the wireless patient member device 300.

Additionally and/or alternatively, in some examples of the disclosure, after each communication between either a wireless patient member device 300 or a wireless staff member device 400 and a wireless network transceiver 232', the position of a wireless patient member device 300 or the position of a wireless staff member device 400 is updated to the position of the wireless network transceiver 232' which returned the acknowledgement to the wireless patient member device 300 or to the wireless staff member device 400.

In some additional and/or alternative examples, by holding the switch 402 of the wireless staff device 400, the same as for activating the receiver 411, or having optionally a separate switch at the wireless staff member device 400, the wireless staff member device 400 transmits a staff alarm signal to the nearest wireless network transceiver or, when the nearest wireless network transceiver is unknown, to a group of wireless network transceivers. From there onwards the wireless staff member device 400 is in alarm state. When the switch 402 is activated again, while the wireless staff member device 400 is still in alarm state, the wireless staff member device 400 transmits a cancellation of the alarm of the wireless staff member device 400 to the nearest wireless network transceiver or, when the nearest wireless network transceiver is unknown, to a group of wireless network transceivers. After receiving the cancellation signal a specific network transceiver (the network transceiver closest to the wireless staff member device 400 receiving the cancellation signal) retums an acknowledgement to the wireless staff member device 400.

Additionally and/or alternatively, in some examples of the disclosure, the alarm signal transmitted from the staff member device 400 to the network may either be with high priority or low priority depending on which switch was affected or for how long the switch was hold.

Additionally and/or alternatively, in some examples of the disclosure, the system may handle the staff alarms at a different higher priority level.

Additionally and/or alternatively, in some examples of the disclosure, the transmitting and receiving of signals as well as switching on and off the transmitters 312, 412 and receivers 311, 411 are controlled by a control unit 313, 413, in each device.

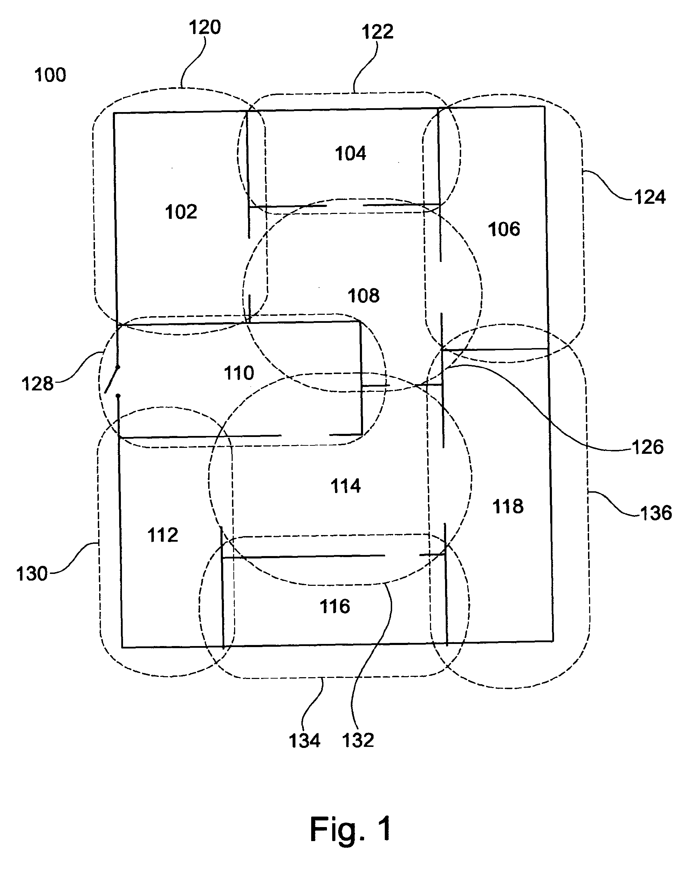

Fig. 1 shows an area in which patient monitoring or patient alarm monitoring or staff alarm monitoring is performed. Such an area may comprise different rooms 102-118. When a wireless monitoring system comprising wireless network transceivers is to be used, transceivers need to be placed so that all of the rooms to be monitored are covered. After placement of the transceivers, the different rooms should be mapped with the transceivers, so that each room is associated with at least one transceiver. A list of this mapping may then be stored and made available to a server. In some examples, transceivers are mapped to rooms 102-118 as illustrated in fig. 1. Each transceiver will in these examples cover a certain zone 120-136 of the monitored area.

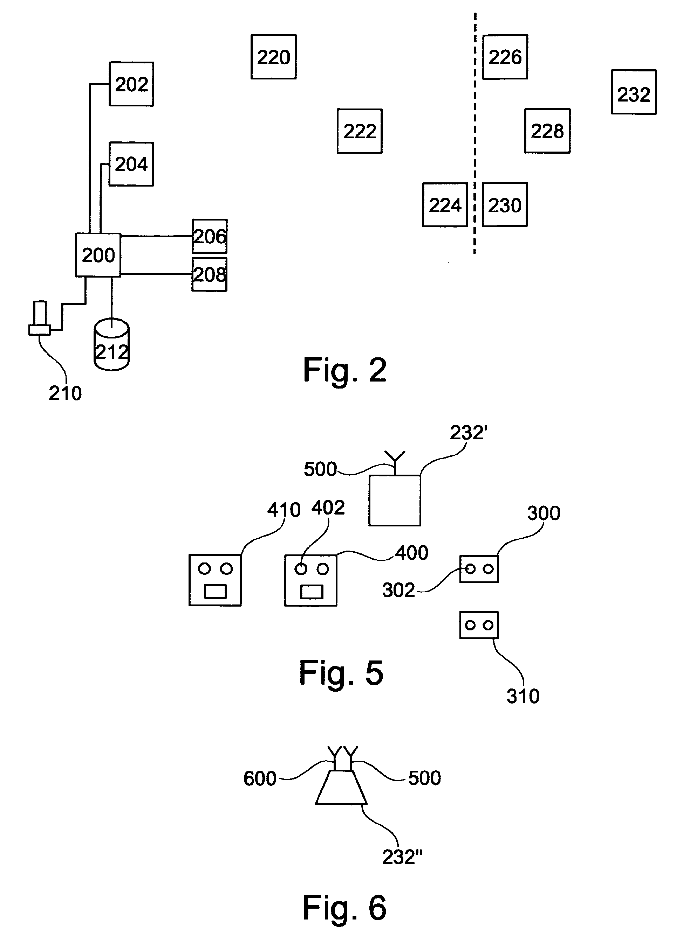

In an example of the disclosure according to Fig. 2, a system comprises a server 200. In fig. 2, the server is connected to overhead screens 206, 208 and to an storage unit 212. Furthermore, the server may also be connected to remote terminals 210 via a network, such as Internet. Moreover, the server is also connected to a first gateway 202 and a second gateway 204. The gateways 202,204 may be in wireless connection with transceivers 220, 222, 224, 226, 228, 230, 232. The gateways 202, 204 are transceivers that communicate with the server 200.

In some examples, a first route, which may be the preferred route of transmitting between the first gateway 202 and a transceiver is selected for each transceiver 220, 222, 224,226,228,230,232. The first route is directly between the first gateway 202 and the transceiver. Alternatively, the first route between the first gateway 202 and the transceiver is via at least one first route intermediate transceiver (as explained below). Whether the first route is direct or via another transceiver depends in one example on a value of signal quality or signal strength, such as a Received Signal Strength Indicator (RSSI), of the transceiver measured at the first gateway or a value of signal quality or signal strength, such as an RSSI, of the first gateway measured at the transceiver. Thus, a route with the best signal quality is set up. In other examples, the selected first route is alternatively or in addition dependent on a Bit Error Rate (BER) and/ or a number of hops.

In some examples, also a second route of transmitting between the first gateway 202 and the transceiver is selected for each transceiver 220,222,224,226,228,230,232. The second route may be a secondary route, which is used only if the first route is not functioning properly. The second route is either directly between the first gateway 202 and the transceiver or between the first gateway 202 and the transceiver via at least one second route intermediate transceiver. Whether the second route is direct or via another transceiver depends on a value of signal quality or signal strength, such as an RSSI, of the transceiver measured at the first gateway or a value of signal quality or signal strength, such as an RSSI, of the gateway measured at the transceiver. However, the at least one second route intermediate transceiver is different from the at least one first route intermediate transceiver. Thus, a redundant route with the second best signal quality is set up. Furthermore, by selecting two different routes, redundancy in the system is ensured.

In some examples, a first route of transmitting between the second gateway 204 and a transceiver is selected for each transceiver 220, 222, 224, 226, 228, 230, 232. The first route is either directly between the second gateway 204 and the transceiver or between the second gateway 204 and the transceiver via at least one first route intermediate transceiver. Whether the first route is direct or via another transceiver depends on a value of a signal strength, such as an RSSI, of the transceiver measured at the second gateway 204 or a value of a signal strength, such as an RSSI, of the second gateway 204 measured at the transceiver. Also a second route of transmitting between the second gateway 204 and the transceiver is selected for each transceiver 220,222,224,226,228,230,232. The second route is either directly between the second gateway 204 and the transceiver or between the second gateway 204 and the transceiver via at least one second route intermediate transceiver, depending on a value of a signal strength, such as an RSSI, of the transceiver measured at the second gateway 204 or a value of a signal strength, such as an RSSI, of the second gateway 204 measured at the transceiver. The at least one second route intermediate transceiver is different from the at least one first route intermediate transceiver. Thus, there are two gateways for redundancy and both gateways have defined preferred and second routes. Thus, if anything happens to the first gateway 202, the second gateway 204 can directly replace the first gateway 202 and there will still be route redundancy in the system.

In some examples, the second gateway 204 is used for transmission instead of the first gateway 202, whenever the first gateway does not function correctly.

A method of setting up a system is also disclosed. The method may comprise mapping rooms, of a group of rooms, with transceivers of a first group of transceivers 220, 222, 224, 226, 228, 230, 232. As can be seen from fig.1, a transceiver covers a certain zone. As an example, one zone may cover a certain room. In the method, a gateway, for example the first gateway 202, is selected as a current node. From the current node, an installer command is broadcasted. The installer command may be "if this message is heard, then respond". After the installer command has been sent, the current node waits for a present group of transceivers to report as a response to the installer command. The transceivers of the present group should not already be linked to the gateway. The transceivers already linked to the gateway are excluded from the present group. The present group of transceivers belongs to the first group of transceivers, i.e. the present group of transceivers is a subgroup of the first group of transceivers. As an example, transceivers 220, 222 and 224 may have responded to the installer command. However, transceivers 226, 228, 230 and 232 may not have responded to the installer command. In the method, signal strength of each transceiver of the present group of transceivers is measured, for example the signal strength of transceivers 220, 222 and 224 may be measured. Measured values of signal strength are compared. The measured and compared values may be Received Signal Strength Indicators (RSSI) of transceivers in the present group of transceivers. For example transceiver 220 may have the largest signal strength or RSSI, transceiver 222 may have fairly large signal strength or RSSI and transceiver 224 may have fairly low signal strength or RSSI. If a criterion is met for a transceiver, a link between the current node and the transceiver is established. Each transceiver of the present group of transceivers is checked against the criterion. A criterion to be used may be if the RSSI of the transceiver is higher than a certain or predetermined threshold. A second criterion that can be used is if the transceiver responded to the installer command or not. Thus, in the example, according to the first criteria or threshold, links between the gateway and for example transceivers 220 and 222 may be established and according to the second criteria (responded), links between the gateway and transceivers 220, 222 and 224 may be established. Furthermore, from the present group of transceivers, the transceiver with the largest RSSI is selected as a current node. For example transceiver 220 may be selected as a current node. Since this transceiver has an established link with the gateway, it can be selected as the current node, and an installer command may be forwarded to this transceiver from the gateway. The installer command may then be rebroadcasted from the current node. Since the current node now likely is closer to the transceivers that did not respond to the first installer command, new transceivers may respond to the rebroadcasted installer command. As an example, transceivers 226, 228 and 230 may respond to this rebroadcasted installer command. The transceivers 226, 228 and 230 will then use transceiver 220 as an intermediate transceiver and links will be established between transceiver 220 and transceivers 226, 228 and 230 in a similar manner as indicated above. This procedure will be repeated until a first route of transmitting from the gateway to each transceiver has been established.

In some examples, the method further comprises measuring a value of signal strength, such as an RSSI, of the transceiver at the first gateway or measuring a value of signal strength, such as an RSSI, of the gateway at the transceiver, for each transceiver. The method may also comprise, selecting a second route of transmitting between the first gateway and the transceiver either directly between the first gateway and the transceiver or between the first gateway and the transceiver via at least one second route intermediate transceiver depending on a value of the measured signal strength, for each transceiver.

A computer-readable medium having embodied thereon a computer program for processing by a computer is also disclosed. The computer program comprises an optional first code segment for mapping a group of rooms with a first group of transceivers. The computer program also comprises a second code segment for selecting a gateway as a current node. The computer program further comprises a third code segment for from the current node broadcasting an installer command. Furthermore, the computer program comprises a fourth code segment for with the current node waiting for a present group of transceivers, transceivers of the present group not being linked to the gateway, the present group of transceivers belonging to the first group of transceivers, to report as a response to the installer command. Moreover, the computer program comprises a fifth code segment for receiving from the server 200 a measured signal strength of each transceiver of the present group of transceivers. The signal strength is measured by the transceiver 232' and a signal indicative of the signal strength is sent from the transceiver 232' to the server 200. In addition, the computer program comprises a sixth code segment for comparing measured values of signal strength, such as Received Signal Strength Indicators (RSSI), of transceivers in the present group of transceivers. Additionally, the computer program comprises a seventh code segment for, for each transceiver of the present group of transceivers, establishing a link between the current node and the transceiver of the present group of transceivers if a criterion, such as if the RSSI of the transceiver is higher than a threshold, is met. The method also comprises an eighth code segment for from the present group of transceivers, selecting a transceiver with the largest RSSI as a current node. The method may further comprise a ninth code segment for repeating until a first route of transmitting from the gateway to each transceiver has been established.

Optionally, the method may comprise a tenth code segment for measuring, for each transceiver, a value of a signal strength, such as an RSSI, of the transceiver at the first gateway or measuring, for each transceiver, a value of a signal strength, such as an RSSI, of the gateway at the transceiver and an eleventh code segment for selecting, for each transceiver, a second route of transmitting between the first gateway and the transceiver either directly between the first gateway and the transceiver or between the first gateway and the transceiver via at least one second route intermediate transceiver depending on the value of signal strength.

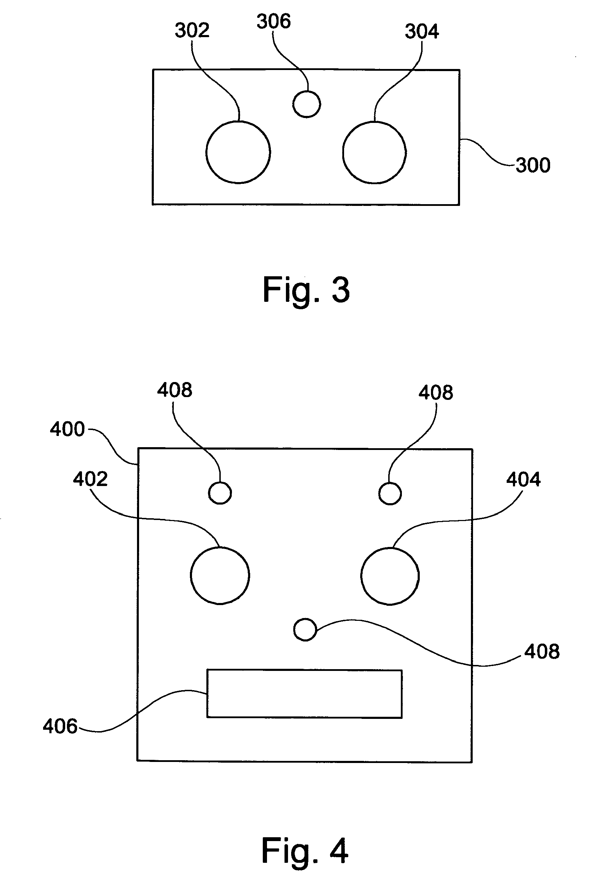

In some examples, a transceiver of the plurality of transceivers can be replaced with a new transceiver by plug and play. Thus, the system can self-heal if a transceiver needs to be replaced, and thus no physical device configuration or user intervention is needed during replacement of a transceiver. In some examples, the system also comprises one or several patient member devices 300, each specific to a patient. These patient member devices 300 are intended to be used by specific patients, i.e. each patient to be monitored is given a personal patient member device 300. A patient member device 300 is illustrated in fig. 3. The patient member device 300 may have a first switch, which may be a button 302 or an area to press on a touch screen, i.e. a selection area of a touch screen, and a second switch, which may be a button 304 or an area to press on a touch screen. The first switch or button 302 may be used for sending an alarm, i.e. the first switch, such as button, 302 may be used for emergencies. Thus, the switch may be used for triggering an alarm and may therefore be an alarm switch. The second switch, such as button, 304 may be used for alerting the staff that some form of not urgent assistance is needed, i.e. the second switch, such as button, 304 may be used for situations that would not be classified as emergencies. The patient member device 300 may further have at least one LED 306 for indication of certain events, such as alarm sent and/or alarm acknowledged. Each patient member device 300 has a unique ID, which ID is linked to a specific patient. The linking data is preferably kept in a server database. The server database may be located in the storage unit 212. Thus, there is no programming required at the patient member devices 300. Consequently, ease of use is achieved in mapping and altering the mapping of patient member devices 300 to patients. In some examples, the system also comprises one or several staff member devices 400. Each of these staff member devices 400 are intended to be used by a specific staff members, such as physicians, nurses, care givers or janitors. Each staff member or at least a plurality of staff members is provided with a personal staff member device 400. A staff member device 400 is illustrated in fig. 4. The staff member device 400 may have a first switch, which may be a button 402 or an area to press on a touch screen and a second switch, which may be a button 404 or an area to press on a touch screen. In one example, the staff member device 400 only has one switch, such as button, 402. The single switch, such as button, 402 facilitates that the device 400 can be operated without looking at it as there is only one switch, such as button, to press. Therefore the staff member device 400 can be carried in a pocket of a staff member. This is advantageous, since staff members should not wear anything around their arms, while attending to patients and -1491653948 cancelling patient alarms. The first switch, such as button, 402 may be used for acknowledgment and/or cancellation of an alarm, for example the first switch, such as button, 402 may be used for acknowledging or cancelling emergencies. The second switch, such as button, 404 may be used as a personal alarm switch, such as a button, for example for alerting other staff members that the staff member carrying the staff member specific device 400 is at danger and/or requires help. A transceiver 232' may convey the alarm signal to the server 200 with a signal. The server 200 or a controller function thereof then sends a control signal to the plurality of overhead screens 206, 208. As a response to this control signal the personal alarm is annunciated audibly and/or visually at the overhead screens. One of the first and second switches, such as buttons, 402, 404, for example the first switch, such as button, 402, may also be utilized for another function. Such another function may be updating of the staff member device's position. This may be useful, for example if a staff member is in danger and running or moving away from the danger. The another function may instead or in addition be a personal alarm, i.e. an alarm for alerting other staff members that the staff member carrying the staff member device 400 is at danger and/or requires help. One way of providing the first switch, such as button, 402 with a dual function is to provide the switch, such as button, with a first function if it is pressed shortly, i.e. pressed down during a period that is shorter than a certain time interval, for example 4 seconds, and then provide the switch, such as button, with a second function if the switch, such as button, is pressed down for a time period that is longer than a certain time interval, such as 4 seconds. The staff member device 400 may further have at least one LED 408 for indication of certain events, such as acknowledgment received by a patient member device.

Furthermore, the staff member device may be equipped with a display for displaying relevant information, such as information related to a patient requiring assistance. Such patient member information may be advantageous to have, since it may facilitate assistance given to the patient, for example the staff member assisting the patient may be given information that will make it easier and/or faster for the staff member to decide what kind of assistance to give. In some examples, staff member device 400 is able to give audible feedback for example when the patient device alarm is cancelled. An audible feedback is advantageous, since the staff member will not be distracted, when attending to a patient and acknowledging the patient alarm. The staff member device 400 may be provided with a loudspeaker or a buzzer for giving audible feedback to the staff member.

The system can keep a record of the alarm- or alert time and the event duration of all patient- and staff alarms. This record is kept at the central database and may be used to add to the staff planning schedules. The central database may be located in the storage unit 212. It may be advantageous to identify exactly which member of staff attended to the patient and/or which member of staff created a high priority staff alarm. This is possible, since each staff member device 400 has a unique ID, which ID may be linked to a specific staff member. In such case, mapping of staff member devices 400 to individual members of staff may be used. Such mapping occurs at the server 200 and does not require any programming of the staff member devices 400. Consequently, ease of use is achieved in mapping and altering the mapping of staff member devices 400 to staff members.

Fig. 5 shows some of the units of one example of the disclosure. One of the units is a transceiver 232'. The transceiver 232' has in this example an antenna 500 for transmitting and receiving signals from other units, such as patient member devices 300 and staff member devices 400, of the system. In some examples, signals between a patient member device 300 and a staff member device 400 may be conveyed via transceiver 232'. In other examples, signals between a patient member device 300 and a staff member device 400 may be sent directly between the devices 300, 400. It is also possible that some signals are sent between a patient member device 300 and a staff member device 400 directly and some signals between a patient member device 300 and a staff member device 400 via transceiver 232'.

In some examples, illustrated in Fig. 6, the transceiver 232' has a first antenna 500 and a second antenna 600. The antennas 500,600 are separated with a certain distance. Separation of antennas with a certain distance is also known as spatial diversity. In one example, spatial diversity is achieved by switching between two or more antennas. In another example, spatial diversity is achieved by using multiple transceivers, each transceiver having an antenna. This example is advantageous, since retransmission of the signal when the weaker antenna has been in effect is avoided. Consequently, this example is beneficial, because the reliability of the system is improved and the battery life of the portable devices is prolonged.

By the use of two antennas, the transceiver will be more reliable, since even if one of the antennas does not function properly, the transceiver 232' will still be able to transmit and receive signals. Furthermore, by choosing a separation distance of the antennas in a certain way, dead-spots, i.e. places where no signal can be obtained, can be avoided, since the physical distance between the antennas is related to the carrier frequency.

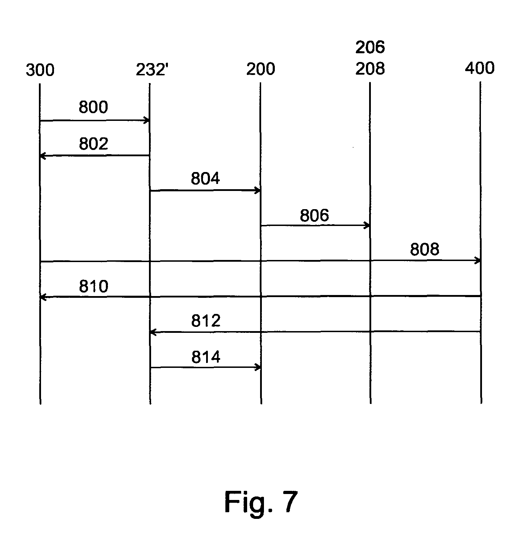

In one example of the disclosure according to Fig. 7, an alarm signal 800 will be broadcasted to a network of fixed transceivers 232' from a patient member device 300 as a response to an event, where a patient has pressed an alarm switch, such as a button. The transceiver 232' having the best signaling path, typically the transceiver closest to the patient member device 300, will respond first.

In some examples, the patient member devices 300, 310 and/or the staff member devices 400, 410 and/or the transceivers are wireless devices. In these examples, the listen-before-talk technique, which is part of the CSMA-CA messaging protocol between all wireless devices, prevents other transceivers 232' from responding to the same patient alarm message.

As a response to this signal, the transceiver 232' will transmit an acknowledgement signal 802 back to the patient member device 300.

At approximately the same time, the transceiver 232' will convey the alarm signal to the server 200 with a signal 804. The server 200 or a controller function thereof sends a control signal 806 to the plurality of overhead screens 206, 208. As a response to this control signal the patient alarm is annunciated audibly and/or visually at the overhead screens 206, 208. A staff member will hear and/or see the alarm and attend to the patient, who sent the alarm. When the staff member, carrying the staff member device 400, brings the staff member device 400 close enough, for example closer than a threshold value, such as 1 m, 3m or 5m, to the patient member device 300, the alarm can be cancelled by the staff member pressing a switch, such as button, such as the first switch, such as button, 402 of the staff member device 400. The threshold value may be a predetermined value. In some examples, the threshold value is adjusted by reduction of the transmit levels of the patient member devices 300 and/or the staff member devices 400. In one example, when the staff member is within close range, i.e. closer than the threshold value, of the patient, who sent the alarm, the staff member may momentarily press the switch, such as button, 402 of the staff member device 400. This will open up a receiving time window, during which the staff member device 400 can receive an alarm-cancellation request 808 from a patient member device 300, i.e. a receiver of the staff member device 400 is activated for a limited time. This receiver may be referred to as a first receiver. Within the staff member device receiving time window, which may be between 1-30 seconds and preferably around 5 or 6 seconds, the staff member presses the switch, such as button, 302 of the patient member device 300, which has sent the alarm. The patient member device 300 will as a response to the switch, such as button, 302 being pressed, send a request 808 to cancel the patient alarm to the staff member device 400. This is performed at reduced RF range. This request 808 is received by the staff member device 400, if it is sent within the staff member device receiving time window as long as the patient member device 300 and the staff member device are closer to each other than the threshold value. The staff member device 400 replies to the patient member device 300 with an acknowledgement signal 810 to cancel the patient alarm. As a response to this acknowledgement signal, the patient member device 300 exits an alarm state and can again send an alarm into the system. The staff member device 400 now transmits a cancellation message 812 to a transceiver 232', which transceiver 232' will convey 814 the cancellation message to the server 200. The patient alarm is then cancelled at the server 200. Thereafter the annunciation of the alarm is cleared and when no more alarms are present, the audible indication is stopped. In this example, the staff member device 400 will not receive any alarms. The staff member device 400 is used to cancel alarms from patient member devices 300 and/or other staff member devices 400 by direct communication with the device in question. Furthermore, most of the communication is in this example performed by the staff member devices 400 and not by the patient member devices 300. This is advantageous, since the staff member device 400 has much more battery capacity than the patient member device 300. Moreover, the staff member device receiving time window can be made sufficiently short and the transmit levels of the patient member devices 300 and/or the staff member devices 400 can be reduced sufficiently to assure that only the intended patient alarm, and thus not any alarms from patients nearby, is acknowledged.

In some examples, it is ensured that cancellation of an alarm can only be made when the staff member device 400 is within a certain distance of the patient member device 300 by line of sight, for example by using an IR emitter and an IR receiver, which are operated as part of the patient alarm cancellation method. In these examples, the patient member device 300 has an IR emitter and the staff member device 400 has an IR receiver. Alternatively the patient member device 300 has an IR receiver and the staff member device 400 has an IR emitter.

In some examples, it is ensured that cancellation of an alarm can only be made when the staff member device 400 is within a certain distance of the patient member device 300 by detection of a magnetic field emitted by a device, such as a magnet or a coil, with a corresponding device, such as a reed switch or a hall sensor. In these examples, the patient member device 300 has a magnet or a coil and the staff member device 400 has a reed switch or a hall sensor. Alternatively the patient member device 300 has a reed switch or a hall sensor and the staff member device 400 has a magnet or a coil.

In some examples, it is ensured that cancellation of an alarm can only be made when the staff member device 400 is within a certain distance of the patient member device 300 by emission of an RF field, for example by the staff member device 400, automatically or after pressing a switch, such as button, which is detected at the patient member device 300 and/or powers a circuit at the patient member device 300.

In some examples, it is ensured that cancellation of an alarm can only be made when the staff member device 400 is within a certain distance of the patient member device 300 by emission of an RF field, for example by the staff member device 400, where the emitting device detects and/or communicates with a RFID tag, which is part of the patient member device 300. Alternatively, emission of an RF field by the patient member device 300 is detected by a RFID tag, which is part of the staff member device 400.

In some examples, it is ensured that cancellation of an alarm can only be made when the staff member device 400 is within a certain distance of the patient member device 300 by using NFC techniques.

In some examples, routing tables for the first route and the second route are established during setup of the system. The routing tables are thereafter downloaded into each transceiver 232'. Thus, the transceivers 232' can perform the first routing, which may be a preferred routing and/or the second routing, which may be redundant, retry autonomously.

In some examples, all communication to and/or from the staff member device 400 and to and/or from patient member device 300 is initiated at the device itself. Such initiation may be performed automatically, for example by sending supervision messages for monitor presence, battery condition and trouble conditions. Alternatively or in addition, such initiation may be performed manually by pressing a switch, such as button, at the device.

In some examples, when the alarm-cancellation request 808 has been sent by the patient member device 300, and if no acknowledgement signal 810 is received from a nearby staff member device 400, the patient member device 300 reverts back to the primary function of the switch, such as button, used, which is typically sending a low or high priority alert message into the system. The priority of transmission of a signal may depend on the switch used. Alternatively, the priority of transmission of a signal may depend on the function activated. This assures that derailing of the communication will not block sending alarms from other patient member devices 300 or from staff member devices 400, and thus automatically brings the system back into synchronization. Such situations may occur for example when staff member devices 400 are momentarily out of range while alarm/alert messages are being sent.

In other examples, for example when no staff member device 400 is present or if the receiver of the staff member device 400 was shut down due to a timeout, the patient member device 300 may repeat transmission of the patient alarm into the network, for example to the overhead screens 206,208 via a transceiver 232' and the server 200.
The staff alarm may function similar to a patient alarm. If a staff member needs assistance either with a patient or being attacked, the staff member may affect a switch on the staff member device to transmit a staff alarm signal to the network. The staff alarm signal may either be send with high or low priority to the nearest wireless network transceiver or, when the nearest wireless network transceiver is unknown, to a group of wireless network transceivers. The priority of transmission of a signal may depend on the switch used. Alternatively, the priority of transmission of a signal may depend on the function activated. From there onwards the wireless staff member device is in alarm state. When the switch is activated, while the wireless staff member device is still in alarm state, the wireless staff member device transmits a cancellation of the alarm of the wireless staff member device to the nearest wireless network transceiver or, when the nearest wireless network transceiver is unknown, to a group of wireless network transceivers. Thereafter a specific network transceiver (the network transceiver receiving the cancellation signal) returns an acknowledgement to the wireless staff member device.

Additionally and/or alternatively, in some examples of the disclosure, the alarm signal transmitted from the staff member device to the network may either be transmitted with high priority or low priority depending on which switch was affected and/or for how long the switch was hold.

Fig. 8A and 8B illustrates some examples, either of the wireless staff member device 400 and the wireless patient member device 300 or both have a control unit 313,413. The control unit 413 of the wireless staff member device 400 may then be configured to, upon affecting one of the at least one switch 402, activate the receiver 411 to receive a first signal within a range less than a threshold distance during a predetermined activation time, the control unit 413 is further configured to transmit the second signal by the transmitter 412 after receiving the first signal by the receiver 411 within the predetermined activation time. The control unit 413 of the wireless staff member device 400 may in addition or alternatively be configured to periodically transmit a position signal to the wireless network transceiver, such as a supervision signal.
Upon reception of any signal from a wireless staff member device 400 transmitted to the wireless transceiver network, or any signal from a wireless patient device 300 transmitted to the wireless transceiver network, the position of the wireless staff member device 400 or the wireless patient member device 300is updated to the position of the wireless network transceiver nearest to the wireless staff member device 400 or the wireless patient device 300. The updating is preferably performed at a server 200 in the network. Additionally and/or alternatively, in some examples of the disclosure, after an alarm signal has been sent by either a staff member device 400 or a patient member device 300 the periodically sent positioning signal, such as a supervision signal from that specific device is transmitted at a higher rate to the wireless transceiver network. For example, if not in an alarm state a supervision message may be transmitted once every minute, such as every 10 minutes, such as every 30 minutes, such as every hour. When a wireless patient member device or wireless staff member device is in an alarm state the period for transmitting a supervision signal to the wireless transceiver network may be as high as every 10 seconds, such as every 5 seconds, such as every 2 seconds or such as every second.

Additionally, in some examples of the disclosure, the position of the wireless staff member device or wireless patient member device in an alarm state may be continuously updated on an overhead screen.

The control unit 413 of the wireless staff member device 400 may also be configured to cancel an indication of an alarm, activated by the wireless patient member device 300, by transmitting a cancellation signal by the transmitter 412 to the network. The control unit 313 of the wireless patient member device 300 may be configured to indicate the alarm in a network by transmitting an alarm signal by the transmitter 312 to a wireless network transceiver of a group of wireless network transceivers being part of the network, the wireless network transceiver being the wireless network transceiver closest to the wireless patient member device 300.

In some examples, the system is configured to activate an alarm, upon affecting one of the at least one second switch 302 a first time. The system may in these examples further be configured to, upon affecting one of the at least one first switch 402, activate the first receiver 411 to receive the first signal within a range less than a threshold distance during a predetermined activation time; and to transmit the first signal from the second transmitter 312, upon affecting one of the at least one second switch 302 a second time; and to transmit the second signal by the first transmitter 412, after receiving the first signal by the first receiver 411 within the predetermined activation time; and to cancel the alarm after receiving the second signal by the second receiver 311.

In some examples, the method may include some or all of the following steps:
activating a receiver of a wireless staff member device to receive a first signal within a threshold distance from a wireless patient device (300) for a predetermined activation time, by affecting a switch of the wireless staff member device;
activating a transmitter of the wireless patient member device (300) to transmit the first signal by affecting a switch (302) of the wireless patient device (300);
transmitting a second signal from a transmitter of the wireless staff member device when the receiver of the wireless staff member device is receiving the first signal within the predetermined activation time; and
cancelling an alarm activated by the wireless patient member device (300) when the receiver of the wireless patient device (300) is receiving the second signal. The staff device may transmit a message into the network, i.e. to a server via one or more transceivers, to cancel the indication of the patient alarm.

Below is a list of some further examples of this disclosure:
1. A system comprising:
   a first gateway (202);
   optionally a second gateway (204);
   a plurality of transceivers (220, 222, 224, 226, 228, 230, 232); and
   wherein a first route of transmitting between the first gateway (202) and a transceiver is selected for each transceiver (220, 222, 224, 226, 228, 230, 232), the first route being either directly between the first gateway (202) and the transceiver or between the first gateway (202) and the transceiver via at least one first route intermediate transceiver depending on a value of signal quality or signal strength, such as a Received Signal Strength Indicator (RSSI), of the transceiver measured at the first gateway (202) or a value of signal quality or signal strength, such as an RSSI, of the first gateway (202) measured at the transceiver. The system may be a system or a network system.
2. The system of example 1, wherein a second route of transmitting between the first gateway (202) and the transceiver is selected for each transceiver (220, 222, 224, 226, 228, 230, 232), the second route being either directly between the first gateway (202) and the transceiver or between the first gateway (202) and the transceiver via at least one second route intermediate transceiver depending on a value of signal quality or signal strength, such as an RSSI, of the transceiver measured at the first gateway (202) or a value of signal quality or signal strength, such as an RSSI, of the first gateway (202) measured at the transceiver and
   wherein the at least one second route intermediate transceiver is different from the at least one first route intermediate transceiver.
3. The system of example 2, further comprising the second gateway (204), and
   wherein a first route of transmitting between the second gateway (204) and a transceiver is selected for each transceiver (220, 222, 224, 226, 228, 230, 232), the first route being either directly between the second gateway (204) and the transceiver or between the second gateway (204) and the transceiver via at least one first route intermediate transceiver, depending on a value of signal quality or signal strength, such as an RSSI, of the transceiver measured at the second gateway (204) or a value of signal quality or signal strength, such as an RSSI, of the second gateway (204) measured at the transceiver; and
   wherein a second route of transmitting between the second gateway (204) and the transceiver is selected for each transceiver (220, 222, 224, 226, 228, 230, 232), the second route being either directly between the second gateway (204) and the transceiver or between the second gateway (204) and the transceiver via at least one second route intermediate transceiver, depending on a value of signal quality or signal strength, such as an RSSI, of the transceiver measured at the second gateway (204) or a value of signal quality or signal strength, such as an RSSI, of the second gateway (204) measured at the transceiver, the at least one second route intermediate transceiver being different from the at least one first route intermediate transceiver.
4. The system of example 3, wherein the second gateway (204) is used for transmission instead of the first gateway (202), whenever the first gateway (202) does not function correctly.
5. The system of any of the proceeding examples, wherein at least one transceiver has two antennas (500, 600).
6. The system of any of examples 2-5, wherein the first route and/or the second route are utilized for transmission of alarms and/or acknowledgement messages and/or cancellation of the alarms.
7. The system of any of the proceeding examples, further comprising a wireless patient member device (300) and/or a wireless staff member device (400) and/or wherein the transceivers (220, 222, 224, 226, 228, 230, 232) are wireless network transceivers.
8. The system of any of examples 2-7, further comprising:
   a server (200);
   an overhead screen (206); and
   wherein the first and second gateways (202, 204) are connected to the server (200) and wherein the server (200) is connected to the overhead screen (206).
9. The system of any of the proceeding examples, wherein a transceiver of the plurality of transceivers (220, 222, 224, 226, 228, 230, 232) can be replaced with a new transceiver by plug and play.
10. A method of setting up a system comprising:
   a) optionally mapping rooms, of a group of rooms, with transceivers of a first group of transceivers;
   b) selecting a gateway as a current node;
   c) from the current node broadcasting an installer command;
   d) with the current node waiting for a present group of transceivers, transceivers of the present group not being linked to the gateway, the present group of transceivers belonging to the first group of transceivers, to report as a response to the installer command;
   e) measuring a value of signal quality or signal strength of each transceiver of the present group of transceivers;
   f) comparing measured values of signal quality or signal strength, such as Received Signal Strength Indicators (RSSI), of transceivers in the present group of transceivers;
   g) for each transceiver of the present group of transceivers, establishing a link between the current node and the transceiver of the present group of transceivers if a criterion, such as if the RSSI of the transceiver is higher than a threshold, is met;
   h) from the present group of transceivers, selecting a transceiver with a best signal quality or a largest signal strength, such as a largest RSSI, as a current node;
   i) repeating steps c) to h) until a first route of transmitting from the gateway to each transceiver has been established. The method may be used in a system according to any of examples 1-9.
11. The method of example 10, further comprising:
   j) for each transceiver, measuring a value of signal quality or signal strength, such as an RSSI, of the transceiver at the gateway or measuring a value of signal quality or signal strength, such as an RSSI, of the gateway at the transceiver;
   k) for each transceiver, selecting a second route of transmitting between the gateway and the transceiver either directly between the gateway and the transceiver or between the gateway and the transceiver via at least one second route intermediate transceiver depending on a value of the measured signal quality or signal strength.
12. A computer-readable medium having embodied thereon a computer program for processing by a computer, the computer program comprising:
   an optional first code segment for mapping a group of rooms with a first group of transceivers;
   a second code segment for selecting a gateway as a current node;
   a third code segment for broadcasting an installer command from the current node;
   a fourth code segment for, with the current node, waiting for a present group of transceivers, transceivers of the present group not being linked to the gateway, the present group of transceivers belonging to the first group of transceivers, to report as a response to the installer command;
   a fifth code segment for receiving from the server 200 a measured value of signal quality or signal strength of each transceiver of the present group of transceivers;
   a sixth code segment for comparing measured values of signal quality or signal strength, such as Received Signal Strength Indicators (RSSI), of transceivers in the present group of transceivers;
   a seventh code segment for, for each transceiver of the present group of transceivers, establishing a link between the current node and the transceiver of the present group of transceivers, if a criterion, such as if the RSSI of the transceiver is higher than a threshold, is met;
   an eighth code segment for, from the present group of transceivers, selecting a transceiver with a best signal quality or a largest signal strength, such as a largest RSSI, as a current node;
   a ninth code segment for repeating code segments until a first route of transmitting from the gateway to each transceiver has been established. The computer-readable medium having embodied thereon a computer program for processing by a computer may be used in a system according to any of examples 1-9.
13. The computer-readable medium having embodied thereon a computer program for processing by a computer of example 12, the computer program further comprising:
   a tenth code segment for measuring, for each transceiver, a value of signal quality or signal strength, such as an RSSI, of the transceiver at the gateway or measuring, for each transceiver, a value of signal quality or signal strength, such as an RSSI, of the gateway at the transceiver;
   an eleventh code segment for selecting, for each transceiver, a second route of transmitting between the gateway and the transceiver either directly between the gateway and the transceiver or between the gateway and the transceiver via at least one second route intermediate transceiver depending on the value of signal quality or signal strength.

As used herein, the singular forms "a", "an" and "the" are intended to include the plural forms as well, unless expressly stated otherwise. It will be further understood that the terms "includes," "comprises," "including" and/or "comprising," when used in this specification, specify the presence of stated features, integers, steps, operations, elements, and/or components, but do not preclude the presence or addition of one or more other features, integers, steps, operations, elements, components, and/or groups thereof. It will be understood that when an element is referred to as being "connected" or "coupled" to another element, it can be directly connected or coupled to the other element or intervening elements may be present. Furthermore, "connected" or "coupled" as used herein may include wirelessly connected or coupled. As used herein, the term "and/or" includes any and all combinations of one or more of the associated listed items.

Unless otherwise defined, all terms (including technical and scientific terms) used herein have the same meaning as commonly understood by one of ordinary skill in the art to which this disclosure belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein. The present disclosure has been described above with reference to specific examples. However, other examples than the above described are equally possible within the scope of the disclosure. Different method steps or a different order thereof than those described above may be provided within the scope of the disclosure. The different features and steps of the disclosure may be combined in other combinations than those described. The scope of the disclosure is only limited by the appended patent claims.

## Claims

1. A system for patient-staff acknowledgment, including:
a wireless staff member device (400), such as any of claims 10 to 13, including:
a first receiver (411) for receiving a first signal from a wireless patient member device;
a first transmitter (412) for transmitting a second signal to said wireless patient member device; and
at least one first switch (402);
a wireless patient member device (300), such as of claim 14, including:
at least one second switch (302);
a second transmitter (312) for transmitting said first signal to said wireless staff member device;
a second receiver (311) for receiving said second signal from said wireless staff member device;
wherein said wireless patient member device is configured to activate an alarm, upon affecting one of said at least one second switch (302) a first time, and to transmit said first signal from said second transmitter (312), upon affecting one of said at least one second switch (302) a second time; said wireless staff member device is configured to, upon affecting one of said at least one first switch (402), activate said first receiver (411) to receive said first signal within a range less than a threshold distance during a predetermined activation time; and to transmit said second signal by said first transmitter (412), after receiving said first signal by said first receiver (411) within said predetermined activation time; and said wireless patient member device is configured to cancel said alarm after receiving said second signal by said second receiver (311).

2. The system of claim 1, wherein said system comprises a wireless network transceiver of a group of wireless network transceivers being part of a network; and/or said threshold distance is a distance between said wireless staff member device (400) and said wireless patient member device (300); and/or said distance is calculated based on a measured position of said wireless patient member device (300) and/or a measured position of said wireless staff member device (400).

3. The system of any of claim 2, wherein said measured position of said wireless patient member device (300) and/or said measured position of said wireless staff member device (400) are based on a Received Signal Strength Indicator (RSSI) signal obtained from said wireless network transceiver of a group of wireless network transceivers, said wireless network transceiver being the wireless network transceiver closest to the wireless patient device (300) and/or the wireless staff member device (400).

4. The system of any of claims 2 or 3, wherein said wireless network transceiver includes two antennas (500, 600), and wherein an antenna with the largest RSSI is selected for position calculations.

5. The system of any of claims 2 to 4, wherein said wireless patient member device is configured to indicate said alarm in said network by transmitting an alarm signal by said second transmitter to a wireless network transceiver of a group of wireless network transceivers being part of said network, said wireless network transceiver being the wireless network transceiver closest to said wireless patient member device; and/or said wireless network transceiver closest to said wireless patient member device is configured to transmit an alarm acknowledgment signal to said wireless patient member device after receiving said alarm signal.

6. The system of any of claim 5, wherein said wireless patient member device is configured to repeatedly transmitting said alarm signal to indicate activation of said alarm to said network by transmitting said alarm signal to said wireless network transceiver being a wireless network transceiver and/or a plurality of transceivers of said group of wireless network transceivers closest to said wireless patient member device.

7. The system of any of claims 2 to 6, wherein said wireless staff member device is configured to acknowledge reception of an alarm from said network by transmitting a first acknowledgment signal by said first transmitter to a wireless network transceiver of a group of wireless network transceivers being part of said network, said wireless network transceiver being a wireless network transceiver closest to said wireless patient device (300) and/or the wireless staff member device (400) or a group of transceivers; and wherein said first acknowledgment signal is optionally acknowledged by a second acknowledgment signal transmitted from said wireless patient device (300) to said wireless network transceiver being a wireless network transceiver and/or a plurality of transceivers of said group of wireless network transceivers closest to said wireless patient device (300) and/or the wireless staff member device (400).

8. The system of any of claims 2 to 7, wherein said wireless staff member device is configured to cancel an indication of said alarm, activated by said wireless patient device, by transmitting a cancellation signal by said first transmitter to a wireless network transceiver of a group of wireless network transceivers being part of said network, said wireless network transceiver being a wireless network transceiver and/or a plurality of transceivers of said group of wireless network transceivers closest to said wireless patient device (300) and/or the wireless staff member device (400).

9. The system of any of claims 2 to 8, wherein said wireless patient member device and/or said wireless staff member device are configured to update its position in said network by transmitting a position signal by said first and/or second transmitter to said network, said updating is preferably performed at a server (200) in said network when said wireless network transceiver closest to wireless patient member device and/or said wireless staff member device receives said position signal.

10. A wireless staff member device (400) for use in a system for patient-staff acknowledgment, including:
a receiver for receiving a first signal from a wireless patient member device, such as any of claims 14;
a transmitter for transmitting a second signal to said wireless patient member device, such as any of claims 14; and
at least one switch (402);
a control unit configured to, upon affecting one of said at least one switch, activate said receiver to receive a first signal within a range less than a threshold distance during a predetermined activation time, said control unit is further configured to transmit said second signal by said transmitter after receiving said first signal by said receiver within said predetermined activation time.

11. The wireless staff member device of claim 10, wherein said threshold distance is less than 5 meter; and/or said predetermined activation time is in the range 2 to 10 seconds, such as 5 seconds; and/or wherein said threshold distance is determined by adjusting a receive sensitivity of said receivers; and/or wherein said second transmitter is configured to transmit said first signal within a range less than a threshold distance by reducing a transmittance level.

12. The wireless staff member device of any of claims 10 to 11, further including a counter configured to count said activation time.

13. The wireless staff member device of any of claims 10 to 12, wherein one of said at least one switch is configured to activate a staff alarm, upon activation of said staff alarm, said control unit is configured to indicate said staff alarm in a network by transmitting a staff alarm signal by said transmitter to a wireless network transceiver of a group of wireless network transceivers being part of said network, said wireless network transceiver being the wireless network transceiver and/or a plurality of transceivers of said group of wireless network transceivers closest to said wireless staff member device.

14. A wireless patient member device for use in a system for patient-staff acknowledgment, including:
at least one switch (302);
a transmitter for transmitting a first signal to a wireless staff member device, such as any of claims 10 to 13;
a receiver for receiving a second signal from said wireless staff member device, such as any of claims 10 to 13;
a control unit configured to, upon affecting one of said at least one switch said first time, activate an alarm, said control unit is further configured to, upon affecting one of said at least one switch a second time, transmit said first signal by said transmitter and cancelling said alarm after receiving said second signal by said receiver as a response to said transmitted first signal.

15. A method of patient-staff acknowledgment, including:
activating a receiver of a wireless staff member device to receive a first signal within a threshold distance from a wireless patient device (300), said receiver being activated for a predetermined activation time, by affecting a switch of said wireless staff member device;
activating a transmitter of said wireless patient member device (300) to transmit said first signal by affecting a switch (302) of said wireless patient device (300);
transmitting a second signal from a transmitter of said wireless staff member device when said receiver of said wireless staff member device is receiving said first signal within said predetermined activation time;
cancelling an alarm activated by said wireless patient member device (300) when said receiver of said wireless patient device (300) is receiving said second signal from said wireless staff member device.

## Patentansprüche

1. System zur Patient-Personal-Bestätigung, umfassend:
drahtlose Vorrichtung für Personalangehörige (400) gemäß einem beliebigen der Ansprüche 10 bis 13, umfassend:
ersten Empfänger (411) für den Empfang eines ersten Signals von einer drahtlosen Vorrichtung für Patientenangehörige;
ersten Sender (412) zur Übertragung eines zweiten Signals an die drahtlose Vorrichtung für Patientenangehörige; und
mindestens einen ersten Schalter (402);
drahtlose Vorrichtung für Patientenangehörige (300) gemäß Anspruch 14, umfassend:
mindestens einen zweiten Schalter (302);
zweiten Sender (312) zur Übertragung des ersten Signals an die drahtlose Vorrichtung für Personalangehörige; und
zweiten Empfänger (311) für den Empfang des zweiten Signals von einer drahtlosen Vorrichtung für Personalangehörige;
wobei die drahtlose Vorrichtung für Patientenangehörige so eingerichtet ist, dass sie bei der einmaligen Betätigung eines von mindestens einem zweiten Schalter (302) einen Alarm aktiviert, und dass sie bei der zweimaligen Betätigung eines von mindestens einem zweiten Schalter (302) das erste Signal von dem zweiten Sender (312) überträgt; wobei die drahtlose Vorrichtung für Personalangehörige so eingerichtet ist, dass sie bei Betätigung eines von mindestens einem ersten Schalter (402) den ersten Empfänger (411) aktiviert, so dass er das erste Signal innerhalb eines Bereiches von weniger als einem Schwellenabstand während einer zuvor festgelegten Aktivierungszeit empfängt; und dass sie das zweite Signal von dem ersten Sender (412) überträgt, nachdem sie das erste Signal von dem ersten Empfänger (411) innerhalb der zuvor festgelegten Aktivierungszeit empfangen hat; und wobei die drahtlose Vorrichtung für Patientenangehörige so eingerichtet ist, dass sie den Alarm widerruft, nachdem sie das zweite Signal von dem zweiten Empfänger (311) empfangen hat.

2. System gemäß Anspruch 1, wobei das System einen drahtlosen Netzwerk-Sendeempfänger aus einer Gruppe von drahtlosen Netzwerk-Sendeempfängern umfasst, welche Teil eines Netzwerkes sind; und/oder wobei es sich bei dem Schwellenabstand um einen Abstand zwischen der drahtlosen Vorrichtung für Personalangehörige (400) und der drahtlosen Vorrichtung für Patientenangehörige (300) handelt; und/oder wobei der Abstand auf der Grundlage einer gemessenen Position der drahtlosen Vorrichtung für Patientenangehörige (300) und/oder einer gemessenen Position der drahtlosen Vorrichtung für Personalangehörige (400) berechnet wird.

3. System gemäß Anspruch 2, wobei die gemessene Position der drahtlosen Vorrichtung für Patientenangehörige (300) und/oder die gemessene Position der drahtlosen Vorrichtung für Personalangehörige (400) auf einem Received-Signal-Strength-Indicator(RSSI)-Signal basiert, das vom drahtlosen Netzwerk-Sendeempfänger aus einer Gruppe von drahtlosen Netzwerk-Sendeempfängern gewonnen wurde, wobei es sich bei dem drahtlosen Netzwerk-Sendeempfänger um den drahtlosen Netzwerk-Sendeempfänger handelt, der sich am nächsten an der drahtlosen Vorrichtung für Patientenangehörige (300) und/oder an der drahtlosen Vorrichtung für Personalangehörige (400) befindet.

4. System gemäß Ansprüchen 2 oder 3, wobei der drahtlose Netzwerk-Sendeempfänger zwei Antennen (500, 600) umfasst, und wobei eine Antenne mit dem stärksten RSSI für die Positionsberechnungen ausgewählt wird.

5. System gemäß einem beliebigen der Ansprüche 2 bis 4, wobei die drahtlose Vorrichtung für Patientenangehörige so eingerichtet ist, dass sie den Alarm in dem Netzwerk durch Übertragung eines Alarmsignals von dem zweiten Sender an einen drahtlosen Netzwerk-Sendeempfänger aus einer Gruppe von drahtlosen Netzwerk-Sendeempfängern, die Teil des Netzwerks sind, anzeigt, wobei es sich bei dem drahtlosen Netzwerk-Sendeempfänger um den drahtlosen Netzwerk-Sendeempfänger handelt, der sich am nächsten an der drahtlosen Vorrichtung für Patientenangehörige befindet; und/oder wobei der drahtlose Netzwerk-Sendeempfänger, der sich am nächsten an der drahtlosen Vorrichtung für Patientenangehörige befindet, so eingerichtet ist, dass er, nachdem er das Alarmsignal empfangen hat, ein Alarm-Bestätigungssignal an die drahtlose Vorrichtung für Patientenangehörige überträgt.

6. System gemäß Anspruch 5, wobei die drahtlose Vorrichtung für Patientenangehörige so eingerichtet ist, dass sie das Alarmsignal wiederholt überträgt, um dem Netzwerk die Aktivierung des Alarms anzuzeigen, was durch Übertragung des Alarmsignals an den drahtlosen Netzwerk-Sendeempfänger erfolgt, bei dem es sich um einen drahtlosen Netzwerk-Sendeempfänger und/oder eine Vielzahl von Sendeempfängern aus einer Gruppe von drahtlosen Netzwerk-Sendeempfängern handelt, die sich am nächsten an der drahtlosen Vorrichtung für Patientenangehörige befindet.

7. System gemäß einem beliebigen der Ansprüche 2 bis 6, wobei die drahtlose Vorrichtung für Personalangehörige so eingerichtet ist, dass sie den Empfang eines Alarms von dem Netzwerk durch die Übertragung eines ersten Bestätigungssignals von dem ersten Sender an einen drahtlosen Netzwerk-Sendeempfänger aus einer Gruppe von drahtlosen Netzwerk-Sendeempfängern, die ein Teil des Netzwerks sind, bestätigt, wobei es sich bei dem drahtlosen Netzwerk-Sendeempfänger um einen drahtlosen Netzwerk-Sendeempfänger, der sich am nächsten an der drahtlosen Vorrichtung für Patientenangehörige (300) und/oder an der drahtlosen Vorrichtung für Personalangehörige (400) befindet, oder um eine Gruppe von Sendeempfängern handelt; und wobei das erste Bestätigungssignal optional durch ein zweites Bestätigungssignal bestätigt wird, welches von der drahtlosen Vorrichtung für Patienten (300) an den drahtlosen Netzwerk-Sendeempfänger übertragen wird, bei dem es sich um einen drahtlosen Netzwerk-Sendeempfänger und/oder eine Vielzahl von Sendeempfängern aus der Gruppe von drahtlosen Netzwerk-Sendeempfängern handelt, die sich am nächsten an einer drahtlosen Vorrichtung für Patientenangehörige (300) und/oder der drahtlosen Vorrichtung für Personalangehörige (400) befindet.

8. System gemäß einem beliebigen der Ansprüche 2 bis 7, wobei die drahtlose Vorrichtung für Personalangehörige so eingerichtet ist, dass sie die Anzeige eines Alarms, welcher von der drahtlosen Vorrichtung für Patientenangehörige aktiviert wurde, durch die Übertragung eines Widerruf-Signals von einem ersten Sender an einen drahtlosen Netzwerk-Sendeempfänger aus einer Gruppe von drahtlosen Netzwerk-Sendeempfängern, welche Teil des Netzwerks sind, widerruft, wobei es sich bei dem drahtlosen Netzwerk-Sendeempfänger um einen drahtlosen Netzwerk-Sendeempfänger und/oder eine Vielzahl von Sendeempfängern aus einer Gruppe von drahtlosen Netzwerk-Sendeempfängern handelt, die sich am nächsten an der drahtlosen Vorrichtung für Patientenangehörige (300) und/oder der drahtlosen Vorrichtung für Personalangehörige (400) befindet.

9. System gemäß einem beliebigen der Ansprüche 2 bis 8, wobei die drahtlose Vorrichtung für Patientenangehörige und/oder die drahtlose Vorrichtung für Personalangehörige so eingerichtet ist, dass sie ihre Position in dem Netzwerk durch Übertragung eines Positionssignals von dem ersten und/oder zweiten Sender an das Netzwerk aktualisieren, wobei die Aktualisierung vorzugsweise an einem Server (200) in dem Netzwerk durchgeführt wird, wenn der drahtlose Netzwerk-Sendeempfänger, der sich am nächsten an der drahtlosen Vorrichtung für Patientenangehörige und/oder der drahtlosen Vorrichtung für Personalangehörige befindet, das Positionssignal empfängt.

10. Drahtlose Vorrichtung für Personalangehörige (400) für den Einsatz in einem System für die Patient-Personal-Bestätigung, umfassend:
Empfänger für den Empfang eines ersten Signals von einer drahtlosen Vorrichtung für Patientenangehörige gemäß Anspruch 14;
Sender zur Übertragung eines zweiten Signals an die drahtlose Vorrichtung für Patientenangehörige gemäß Anspruch 14; und
mindestens einen Schalter (402);
drahtlose Vorrichtung für Patientenangehörige (300) gemäß Anspruch 14, umfassend:
Steuereinheit, die so eingerichtet ist, dass sie bei der Betätigung eines von mindestens einem zweiten Schalter den Empfänger aktiviert, so dass er ein erstes Signal innerhalb eines Bereiches von weniger als einem Schwellenabstand während einer zuvor festgelegten Aktivierungszeit empfängt, wobei die Steuereinheit ferner so eingerichtet ist, dass sie das zweite Signal von dem Sender überträgt, nachdem sie das erste Signal von dem Empfänger innerhalb der zuvor festgelegten Aktivierungszeit empfangen hat.

11. Drahtlose Vorrichtung für Personalangehörige gemäß Anspruch 10, wobei der Schwellenabstand weniger als 5 Meter beträgt; und/oder wobei die zuvor festgelegte Aktivierungszeit im Bereich von 2 bis 10 Sekunden, wie beispielsweise 5 Sekunden, liegt; und/oder wobei der Schwellenabstand durch die Einstellung einer Empfangsempfindlichkeit des Empfängers bestimmt wird; und/oder wobei der zweite Sender so eingerichtet ist, dass er ein erstes Signal innerhalb eines Bereiches von weniger als einem Schwellenabstand durch die Reduktion eines Übertragungspegels überträgt.

12. Drahtlose Vorrichtung für Personalangehörige gemäß Anspruch 10 bis 11, ferner einen Zähler umfassend, der so eingerichtet ist, dass er die Aktivierungszeit zählt.

13. Drahtlose Vorrichtung für Personalangehörige gemäß Anspruch 10 bis 12, wobei einer von mindestens einem Schalter so eingerichtet ist, dass er bei Aktivierung des Personalalarms einen Personalalarm aktiviert, wobei die Steuereinheit so eingerichtet ist, dass sie den Personalalarm in einem Netzwerk anzeigt, was durch die Übertragung eines Personalalarm-Signals von dem Sender an einen drahtlosen Netzwerk-Sendeempfänger aus einer Gruppe von drahtlosen Netzwerk-Sendeempfängern, die Teil des Netzwerkes sind, geschieht, wobei es sich bei dem drahtlosen Netzwerk-Sendeempfänger um einen drahtlosen Netzwerk-Sendeempfänger und/oder eine Vielzahl von Sendeempfängern aus einer Gruppe von drahtlosen Netzwerk-Sendeempfängern handelt, die sich am nächsten an der drahtlosen Vorrichtung für Personalangehörige befindet.

14. Drahtlose Vorrichtung für Patientenangehörige für den Einsatz in einem System zur Patient-Personal-Bestätigung, umfassend:
mindestens einen Schalter (302);
Sender zur Übertragung eines ersten Signals an die drahtlose Vorrichtung für Personalangehörige gemäß einem beliebigen der Ansprüche 10 bis 13;
Empfänger zum Empfang eines zweiten Signals von einer drahtlosen Vorrichtung für Personalangehörige gemäß einem beliebigen der Ansprüche 10 bis 13;
Steuereinheit, die so eingerichtet ist, dass sie bei der einmaligen Betätigung eines von mindestens einem Schalter einen Alarm aktiviert, wobei die Steuereinheit ferner so eingerichtet ist, dass sie bei einer zweimaligen Betätigung eines von mindestens einem Schalter das erste Signal von dem Sender überträgt und den Alarm widerruft, nachdem sie das zweite Signal von dem Empfänger als Antwort auf das übertragene erste Signal empfangen hat.

15. Verfahren der Patient-Personal-Bestätigung, umfassend:
Aktivieren eines Empfängers einer drahtlosen Vorrichtung für Personalangehörige, um ein erstes Signal innerhalb eines Schwellenabstandes von einer drahtlosen Vorrichtung für Patientenangehörige (300) zu empfangen, wobei der Empfänger für eine zuvor festgelegte Aktivierungszeit aktiviert wird, was durch die Betätigung eines Schalters der drahtlosen Vorrichtung für Personalangehörige geschieht;
Aktivierung eines Senders der drahtlosen Vorrichtung für Patientenangehörige (300), um durch Betätigung eines Schalters (302) der drahtlosen Vorrichtung für Patientenangehörige (300) das erste Signal zu übertragen;
Übertragung eines zweiten Signals von einem Sender der drahtlosen Vorrichtung für Personalangehörige, sofern der Empfänger der drahtlosen Vorrichtung für Personalangehörige innerhalb der zuvor festgelegten Aktivierungszeit das erste Signal empfängt;
Widerrufen eines Alarms, welcher durch die drahtlose Vorrichtung für Patientenangehörige (300) aktiviert wurde, sofern der Empfänger der drahtlosen Vorrichtung für Patientenangehörige (300) ein zweites Signal von der drahtlosen Vorrichtung für Personalangehörige erhält.

## Revendications

1. Système d'accusé de réception patient-personnel, incluant :
un dispositif de membre du personnel sans fil (400), selon l'une quelconque des revendications 10 à 13, incluant :
un premier récepteur (411) destiné à recevoir un premier signal provenant d'un dispositif patient sans fil,
un premier transmetteur (412) destiné à transmettre un deuxième signal audit dispositif patient sans fil, et
au moins un premier commutateur (402),
un dispositif patient sans fil (300), selon la revendication 14, incluant :
au moins un deuxième commutateur (302),
un deuxième transmetteur (312) destiné à transmettre ledit premier signal audit dispositif de membre du personnel sans fil,
un deuxième récepteur (311) destiné à recevoir ledit deuxième signal provenant dudit dispositif de membre du personnel sans fil,
dans lequel ledit dispositif patient sans fil est configuré pour activer une alarme, lors d'une pression sur l'un dudit au moins un deuxième commutateur (302) une première fois, et pour transmettre ledit premier signal à partir dudit deuxième transmetteur (312), lors d'une pression sur l'un dudit au moins un deuxième commutateur (302) une deuxième fois, ledit dispositif de membre du personnel sans fil est configuré pour, lors d'une pression sur l'un dudit au moins un premier commutateur (402), activer ledit premier récepteur (411) pour recevoir ledit premier signal dans une portée inférieure à une distance de seuil au cours d'un temps d'activation prédéterminé, et pour transmettre ledit deuxième signal au moyen dudit premier transmetteur (412), après la réception dudit premier signal au moyen dudit premier récepteur (411) au cours dudit temps d'activation prédéterminé, et ledit dispositif patient sans fil est configuré pour annuler ladite alarme après la réception dudit deuxième signal au moyen dudit deuxième récepteur (311).

2. Système selon la revendication 1, dans lequel ledit système comprend un émetteur-récepteur réseau sans fil d'un groupe d'émetteurs-récepteurs réseau sans fil qui font partie d'un réseau, et/ou ladite distance de seuil est une distance entre ledit dispositif de membre du personnel sans fil (400) et ledit dispositif patient sans fil (300), et/ou ladite distance est calculée sur la base d'une position mesurée dudit dispositif patient sans fil (300) et/ou d'une position mesurée dudit dispositif de membre du personnel sans fil (400).

3. Système selon l'une quelconque de la revendication 2, dans lequel ladite position mesurée dudit dispositif patient sans fil (300) et/ou ladite position mesurée dudit dispositif de membre de personnel sans fil (400) sont basées sur un signal d'indicateur d'intensité du signal reçu (RSSI) obtenu à partir dudit émetteur-récepteur réseau sans fil d'un groupe d'émetteurs-récepteurs réseau sans fil, ledit émetteur-récepteur réseau sans fil étant l'émetteur-récepteur réseau sans fil le plus proche du dispositif patient sans fil (300) et/ou du dispositif de membre du personnel sans fil (400).

4. Système selon l'une quelconque des revendications 2 ou 3, dans lequel ledit émetteur-récepteur réseau sans fil inclut deux antennes (500, 600), et dans lequel une antenne avec le plus grand indicateur RSSI est sélectionnée pour des calculs de position.

5. Système selon l'une quelconque des revendications 2 à 4, dans lequel ledit dispositif patient sans fil est configuré pour indiquer ladite alarme dans ledit réseau en transmettant un signal d'alarme au moyen dudit deuxième transmetteur vers un émetteur-récepteur réseau sans fil d'un groupe d'émetteurs-récepteurs réseau sans fil qui font partie dudit réseau, ledit émetteur-récepteur réseau sans fil étant l'émetteur-récepteur réseau sans fil le plus proche dudit dispositif patient sans fil, et/ou ledit émetteur-récepteur réseau sans fil le plus proche dudit dispositif patient sans fil est configuré pour transmettre un signal d'accusé de réception d'alarme audit dispositif patient sans fil après la réception dudit signal d'alarme.

6. Système selon l'une quelconque de la revendication 5, dans lequel ledit dispositif patient sans fil est configuré pour transmettre de manière répétée ledit signal d'alarme pour indiquer une activation de ladite alarme audit réseau en transmettant ledit signal d'alarme audit émetteur-récepteur réseau sans fil qui est un émetteur-récepteur réseau sans fil, et/ou une pluralité d'émetteurs-récepteurs dudit groupe d'émetteurs-récepteurs réseau sans fil les plus proches dudit dispositif patient sans fil.

7. Système selon l'une quelconque des revendications 2 à 6, dans lequel ledit dispositif de membre du personnel sans fil est configuré pour accuser réception d'une alarme provenant dudit réseau en transmettant un premier signal d'accusé de réception au moyen dudit premier transmetteur à un émetteur-récepteur réseau sans fil d'un groupe d'émetteurs-récepteurs réseau sans fil qui font partie dudit réseau, ledit émetteur-récepteur réseau sans fil étant un émetteur-récepteur réseau sans fil le plus proche dudit dispositif patient sans fil (300) et/ou le dispositif de membre du personnel sans fil (400) ou un groupe d'émetteurs-récepteurs, et dans lequel ledit premier signal d'accusé de réception fait optionnellement l'objet d'un accusé de réception au moyen d'un deuxième signal d'accusé de réception transmis dudit dispositif patient sans fil (300) audit émetteur-récepteur réseau sans fil qui est un émetteur-récepteur réseau sans fil, et/ou une pluralité d'émetteurs-récepteurs dudit groupe d'émetteurs-récepteurs réseau sans fil les plus proches dudit dispositif patient sans fil (300) et/ou dudit dispositif de membre du personnel sans fil (400).

8. Système selon l'une quelconque des revendications 2 à 7, dans lequel ledit dispositif de membre du personnel sans fil est configuré pour annuler une indication de ladite alarme, activée par ledit dispositif patient sans fil, en transmettant un signal d'annulation au moyen dudit premier transmetteur vers un émetteur-récepteur réseau sans fil d'un groupe d'émetteurs-récepteurs réseau sans fil qui font partie dudit réseau, ledit émetteur-récepteur réseau sans fil étant un émetteur-récepteur réseau sans fil et/ou une pluralité d'émetteurs-récepteurs dudit groupe d'émetteurs-récepteurs réseau sans fil les plus proches dudit dispositif patient sans fil (300) et/ou du dispositif de membre du personnel sans fil (400).

9. Système selon l'une quelconque des revendications 2 à 8, dans lequel ledit dispositif patient sans fil et/ou ledit dispositif demande de personnel sans fil sont configurés pour mettre à jour leur position dans ledit réseau en transmettant un signal de position au moyen desdits premier et/ou deuxième transmetteurs audit réseau, ladite mise à jour étant de préférence réalisée au niveau d'un serveur (200) dans ledit réseau lorsque ledit émetteur-récepteur réseau sans fil le plus proche dudit dispositif patient sans fil et/ou dudit dispositif de membre du personnel sans fil reçoit ledit signal de position.

10. Dispositif de membre du personnel sans fil (400) à utiliser dans un système d'accusé de réception patient-personnel, incluant :
un récepteur destiné à recevoir un premier signal provenant d'un dispositif patient sans fil, selon l'une quelconque des revendications 14,
un transmetteur destiné à transmettre un deuxième signal audit dispositif patient sans fil, selon l'une quelconque des revendications 14, et
au moins un commutateur (402),
une unité de commande configurée pour, lors d'une pression sur l'un dudit au moins un commutateur, activer ledit récepteur pour recevoir un premier signal dans une portée inférieure à une première distance de seuil au cours d'un premier temps d'activation prédéterminé, ladite unité de commande est en outre configurée pour transmettre ledit deuxième signal au moyen dudit transmetteur après la réception dudit premier signal au moyen dudit récepteur au cours dudit temps d'activation prédéterminé.

11. Dispositif de membre du personnel sans fil selon la revendication 10, dans lequel ladite distance de seuil est inférieure à 5 mètres, et/ou ledit temps d'activation prédéterminé se trouve dans la plage de 2 à 10 secondes, par exemple 5 secondes, et/ou dans lequel ladite distance de seuil est déterminée en ajustant une sensibilité de réception desdits récepteurs, et/ou dans lequel ledit deuxième transmetteur est configuré pour transmettre ledit premier signal dans une portée inférieure à une distance de seuil en réduisant un niveau de transmittance.

12. Dispositif de membre du personnel sans fil selon l'une quelconque des revendications 10 à 11, incluant en outre un compteur configuré pour compter ledit temps d'activation.

13. Dispositif de membre du personnel sans fil selon l'une quelconque des revendications 10 à 12, dans lequel l'un dudit au moins un commutateur est configuré pour activer une alarme de personnel, lors de l'activation de ladite alarme de personnel, ladite unité de commande est configurée pour indiquer ladite alarme de personnel dans un réseau en transmettant un signal d'alarme de personnel au moyen dudit transmetteur à un émetteur-récepteur réseau sans fil d'un groupe d'émetteurs-récepteurs réseau sans fil qui font partie dudit réseau, ledit émetteur-récepteur réseau sans fil étant l'émetteur-récepteur réseau sans fil et/ou une pluralité d'émetteurs-récepteurs dudit groupe d'émetteurs-récepteurs réseau sans fil les plus proches dudit dispositif de membre du personnel sans fil.

14. Dispositif patient sans fil à utiliser dans un système d'accusé de réception patient-personnel, incluant :
au moins un commutateur (302),
un transmetteur destiné à transmettre un premier signal à un dispositif de membre du personnel sans fil, selon l'une quelconque des revendications 10 à 13,
un récepteur destiné à recevoir un deuxième signal provenant dudit dispositif de membre du personnel sans fil, selon l'une quelconque des revendications 10 à 13,
une unité de commande configurée pour, lors d'une pression sur ledit au moins un commutateur une première fois, activer une alarme, ladite unité de commande est en outre configurée pour, lors d'une pression sur l'un dudit au moins un commutateur une deuxième fois, transmettre ledit premier signal au moyen dudit transmetteur et annuler ladite alarme après la réception dudit deuxième signal au moyen dudit récepteur en réponse audit premier signal transmis.

15. Procédé d'accusé de réception patient-personnel, incluant les étapes consistant à:
activer un récepteur d'un dispositif de membre du personnel sans fil pour recevoir un premier signal à une distance de seuil d'un dispositif patient sans fil (300), ledit récepteur étant activé pendant un temps d'activation prédéterminé, avec une pression sur un commutateur dudit dispositif de membre du personnel sans fil,
activer un transmetteur dudit dispositif patient sans fil (300) pour transmettre ledit premier signal par une pression sur un commutateur (302) dudit dispositif patient sans fil (300),
transmettre un deuxième signal provenant d'un transmetteur dudit dispositif de membre du personnel sans fil lorsque ledit récepteur dudit dispositif de membre du personnel sans fil reçoit ledit premier signal au cours dudit temps d'activation prédéterminé,
annuler une alarme activée par ledit dispositif patient sans fil (300) lorsque ledit récepteur dudit dispositif patient sans fil (300) reçoit ledit deuxième signal provenant dudit dispositif de membre du personnel sans fil.
